# EUROPEAN PATENT SPECIFICATION

(11) **EP 1 210 925 B1**
(45) Date of publication and mention of the grant of the patent: **18.06.2008**
(21) Application number: 01309988.2
(22) Date of filing: 29.11.2001
(51) Int. Cl.: A61F 13/533, A61F 13/15

(54) **Disposable diaper**
Wegwerfwindel
Couche-culotte jetable

(30) Priority: 30.11.2000 JP 2000366046
(43) Date of publication of application: 05.06.2002
(73) Proprietor: UNI-CHARM CORPORATION, Shikokuchuo-shi, Ehime-ken (JP)
(72) Inventor: Ohashi, Naoto, Toyohama, Mitoyo, Kagawa-ken 769-1602 (JP); Ono, Yoshio, Toyohama, Mitoyo, Kagawa-ken 769-1602 (JP); Oba, Toru, Toyohama, Mitoyo, Kagawa-ken 769-1602 (JP)
(74) Representative: Parry, Christopher Stephen

(56) References cited:
- EP-A- 0 441 064
- EP-A- 1 023 884
- EP-A- 1 110 528
- WO-A-90/05513
- US-A- 5 451 442

## Description

This invention relates to a disposable diaper for absorption and containment of excrement.

Japanese Utility Model Application Publication No. 1989-141707A describes a disposable diaper having its absorbent pad divided into a plurality of pad sections. Each of the pad sections comprises a mixture of fluff pulp fibers /superabsorbent polymer particles and a topsheet is joined to a backsheet around the pad section so that a groove depressed from the side of the topsheet toward the side of the backsheet may be defined between each pair of the adjacent pad sections.

Japanese Patent Application Publication No. 1997-51913A describes a disposable absorbent undergarment including a liquid-absorbent core formed with a plurality of slits extending through a thickness direction of the core and arranged intermittently in a longitudinal direction of the undergarment as well as in a transverse direction orthogonal to the longitudinal direction so that top- and backsheets may be bonded together along the slits.

With conventional disposable undergarments, the top- and backsheets are joined together at bottoms of the grooves or slits, so the amount of urine flowing into the grooves or slits can be absorbed by the core only through side walls of the grooves or slits. With such undergarment, the surface area of the core available to absorb body fluids is reduced as the width of the groove or slit is enlarged. Consequently, the absorption rate and capacity for body fluids should be correspondingly reduced.

It is an object of this invention to provide a disposable diaper improved so that a width of each groove to be formed on the side of the diaper's topsheet can be enlarged without reduction in the surface area of the core available to absorb body fluids.

The present invention provides the disposable diaper of independent claim 1. The dependent claims specify preferred but optional features.

This invention provides a disposable diaper comprising a liquid-pervious topsheet, a liquid-impervious backsheet and a liquid-absorbent core covered with an absorbent and diffusive sheet and disposed between these two sheets. The core is formed on a side of the topsheet with at least one groove depressed in a direction from a side of the topsheet toward a side of the backsheet and the groove has a bottom and side walls both covered with the topsheet.

The core contains water-absorbent fibers and superabsorbent polymer particles. The water-absorbent fibers and superabsorbent polymer particles are partially disposed between the top- and backsheets along the bottom of the groove.
Fig. 1 is a plan view showing a diaper;
Fig. 2 is a sectional view taken along a line II - II in Fig. 1;
Fig. 3 is a view similar to that in Fig. 1, showing another diaper;
Fig. 4 is a view similar to that in Fig. 2, showing an embodiment of this invention.

Details of a disposable diaper according to this invention will be more fully understood from the description of an embodiment given hereunder with reference to the accompanying drawings.

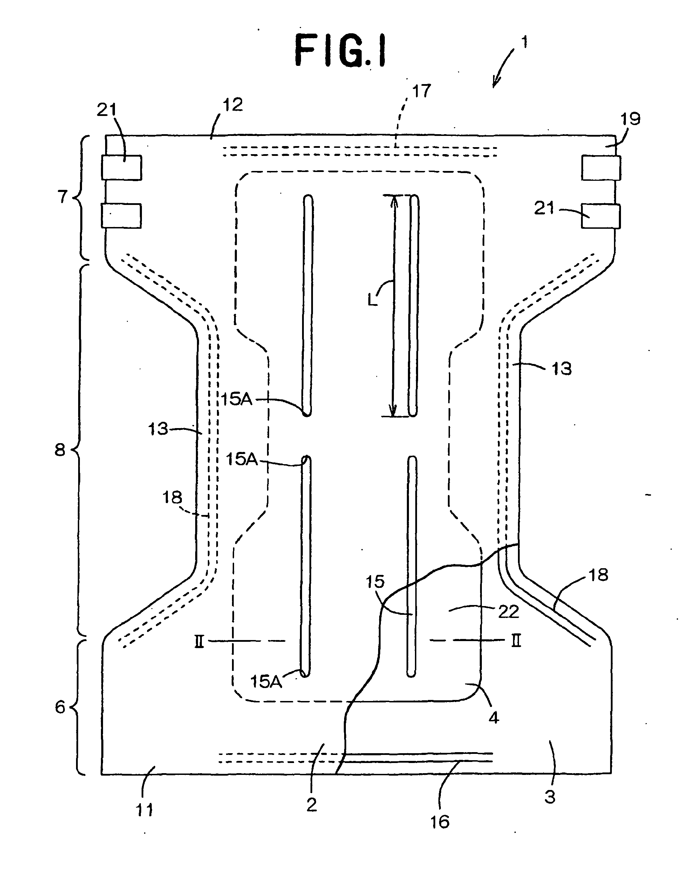

A diaper 1 shown by Fig. 1 in a partially cutaway plan view comprises a liquid-pervious topsheet 2, a liquid-impervious backsheet 3 and a liquid-absorbent core 4 disposed between these two sheets 2, 3. The diaper 1 has a front waist region 6, a rear waist region 7 and a crotch region 8 extending between these two waist regions 6, 7. The top- and backsheets 2, 3 extend outward beyond a peripheral edge of the core 4 and are overlapped to each other and water-tightly joined together over these extensions. Along longitudinally outer end portions 11, 12 of the front and waist regions 6, 7 and transversely opposite side edge portions 13 of the crotch region 8, respectively, elastic members 16, 17 associated with a waist-opening and elastic members 18 associated with leg-openings are secured under tension to the inner surface of the topsheet 2 and/or the backsheet 3. The rear waist region 7 is provided on its transversely opposite side edge portions 19 with tape fasteners 21. On the inner surface of the diaper 1, the core 4 is formed on its transversely opposite side edge portions 22 with grooves 15 extending in parallel to each other in the longitudinal direction of the diaper 1.

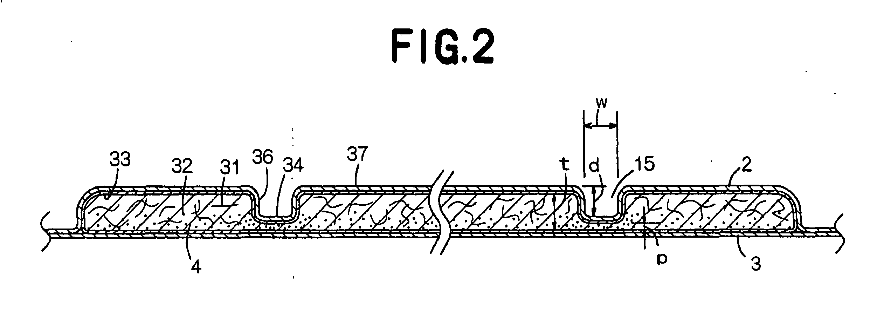

Fig. 2 is a sectional view taken along a line II - II in Fig. 1. The core 4 comprises a mixture of fluff pulp fibers and superabsorbent polymer particles 32 entirely wrapped with an absorbent and diffusive sheet 33 such as tissue paper. The core 4 has a thickness t in a transversely middle zone of the crotch region 8. Along each of the grooves 15, the core 4 is depressed in a direction from the topsheet 2 toward the backsheet 3. The groove 15 has its inner surface covered with the topsheet 2 and the absorbent and diffusive sheet 33 directly underlies the topsheet 2. The groove 15 has a bottom 34 and side walls 36. Referring to Fig. 2, d designates a depth of the groove 15 as measured from a body-side surface 37 of the diaper 1 in the vicinity of the groove 15 to the bottom 34, p designates a thickness of the core 4 as measured from the bottom 34 to the backsheet 3 and w designates a width of the groove 15 as measured at the level of the body-side surface 37.

In such diaper 1, the core 4 preferably has a basis weight of about 200 - 700 g/m², a thickness t of about 2 - 20 mm and contains comminuted pulp by about 98 - 5 % by weight and superabsorbent polymer particles 32 by 2 - 95 % by weight. A density at which the polymer particles 32 are distributed within the core 4 gradually become higher in the thickness direction of the core 4 from the topsheet 2 toward the backsheet 3. The polymer particles 32 are present also in the vicinity of the bottom 34 as well as in the vicinity of the side walls 36 of the groove 15 and partially joined together with the absorbent and diffusive sheet 33 by means of appropriate adhesive such as hot melt adhesive (not shown). Such joining together may be achieved also by compressing at least one of the core 4 and the absorbent and diffusive sheet 33 in desired regions under wet condition. The absorbent and diffusive sheet 33 is joined to the topsheet 2 over the bottoms 34 and the side walls 36 by means of adhesive (not shown). The groove 15 preferably has the width w of about 2 - 20 mm, a length L (See Fig. 1) of at least 20mm and the depth d corresponding to about 10 - 90 % of the thickness t of the core 4. The thickness p of the core 4 as measured from the bottom 34 of the groove 15 to the backsheet 3 corresponds to about 10 - 90 % of the thickness t of the core 4.

Such a unique arrangement of the diaper 1 allows an amount of body fluids, for example, urine flowing on the topsheet 2 transversely of the diaper 1 to be collected in the grooves 15 and thereby to prevent the amount of body fluids from leaking sideways. The amount of body fluids collected in the grooves 15 then spreads in the longitudinal direction of the diaper 1 along the respective grooves 15, and is thereupon absorbed by the core 4 over the bottoms 34 and the side walls 36. A plurality of superabsorbent polymer particles 32 joined to the absorbent and diffusive sheet 33 defining the bottoms 34 as well as the side walls 36 swell as soon as they absorb the body fluids and cohere together so as to form gel block extending along the surface of the respective grooves 15. The presence of such gel block serves to protect the grooves 15 from completely collapsing under a wearer's body weight and, in consequence, having their function impaired. It should be understood here that the gel block functions also to obstruct further permeation of the body fluids into a depth of the core 4. To overcome this problem, it is preferred to distribute the fluff pulp fibers 31 at a density higher than that of the superabsorbent polymer particle 32 in the vicinity of tops of the respective side walls 36 as will be best seen in Fig. 2. In the vicinity of the entire side walls 36, the superabsorbent polymer particles 32 may be mixed with sufficient amount of the fluff pulp fibers 31 so that the fluff pulp fibers 31 may partially extend through the gel block and thereby assist the body fluids to permeate from the grooves 15 further into the depth of the core 4.

As will be apparent from the foregoing description, the core 4 is adapted to absorb the body fluids not only through its flat body-side surface 37 but also through the bottoms 34 and the side walls 36 of the respective grooves 15. This feature can reliably overcome the disadvantage accompanying the diaper of prior art such that the grooves formed by joining the top- and backsheets together necessarily reduces the surface area of the core available to absorb the body fluids. Along the bottoms 34 and the side walls 36 of the core 4, the superabsorbent polymer particles 32 are joined to the absorbent and diffusive sheet 33 so that the polymer particles 32 might not be scattered away far from the vicinity of the grooves 15 even when the grooves 15 are more or less deformed as a wearer's body weight is exerted on the core 4. The core 4 may contain thermoplastic synthetic fibers by 20 % by weight or less and more preferably thermoplastic synthetic fibers having a melting point of 100 °C ±20 °C. When the core 4 is partially heated under a pressure to form the grooves 15, the thermoplastic synthetic resin is molten and deformed to facilitate the formation of the grooves 15. Distribution of the polymer particles 32 in the core 4 may be varied depending on the particular regions of the core 4. For example, a density at which the polymer particles 32 are distributed in the region defined between the transversely adjacent grooves 15, 15 may be adjusted to be higher than that outside the respective grooves 15 to ensure that most of the body fluids is absorbed by the core 4 in the region defined between the grooves 15, 15 and thereby to prevent the body fluids from leaking sideways.

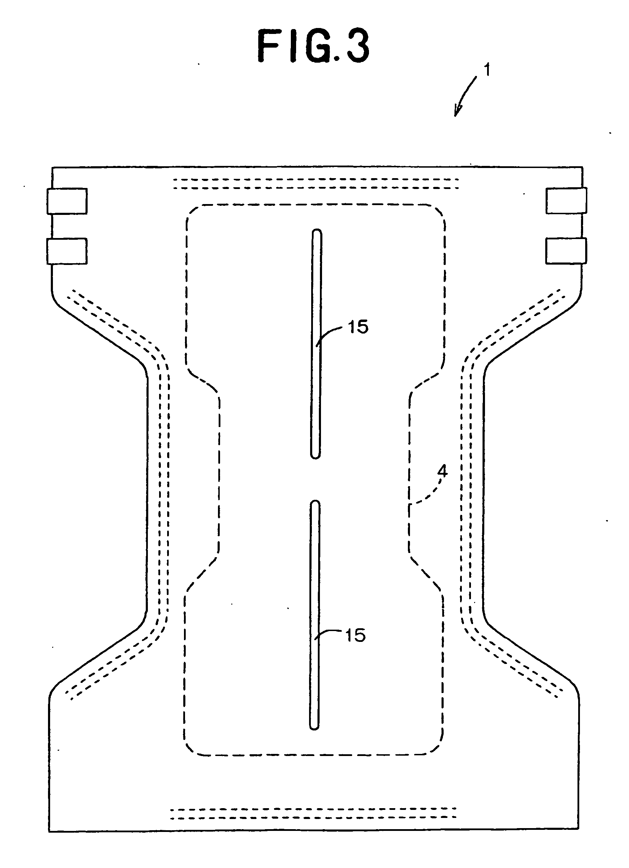

Fig. 3 is a view similar to that in Fig. 1. The diaper 1 shown by Fig. 3 is formed in its transversely middle zone with two grooves 15 extending in the longitudinal direction of the diaper 1. Although these two grooves 15 are illustrated to be spaced apart from each other in the longitudinal direction, if desired. The groove 15 has the same cross-sectional shape as that of the groove 15 illustrated in Fig. 2 and most of the body fluids discharged on the diaper 1 flow in the groove(s) 15 longitudinally extending in the middle zone and then are rapidly absorbed by the core 4 through the bottoms 34 and the side walls 36. In this way, the amount of the body fluids possibly flowing in the transverse direction of the diaper 1 can be correspondingly limited. Therefore, the groove(s) also can prevent the body fluids from leaking sideways of the diaper 1 without reducing the surface area of the core 4 available to absorb the body fluids.

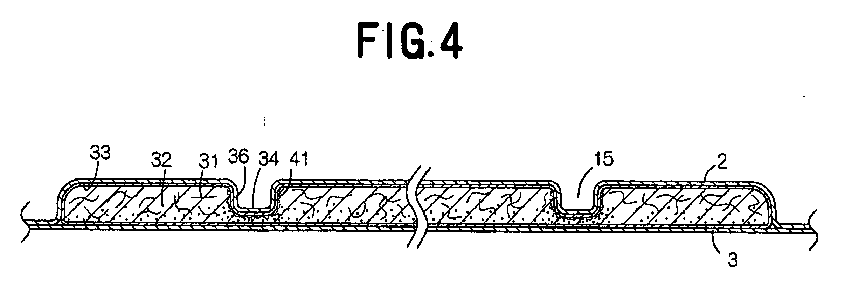

Fig. 4 is a view similar to that in Fig. 2, showing an embodiment of this invention. In the case of this diaper 1, a thin layer 41 of the fluff pulp fibers 31 is closely joined to the inner surface of the absorbent and diffusive sheet 33 along the bottoms 34 and the side walls 36 of the grooves 15. The fluff pulp fibers 31 is distributed at a density higher than its density around the depth of the core 4. The superabsorbent polymer particles 32 are present inside this thin layer 41. With this diaper 1 also, the surface area of the core 4 available to absorb the body fluids is not reduced by the presence of the grooves 15 and the thin layer 41 containing the fluff pulp fibers at high density can accelerate the body fluids flowing into the grooves 15 to be absorbed by the core 4.

The diaper 1 according to this invention is applicable to a baby diaper as well as to an adult diaper. Furthermore, this invention can be implemented not only in the form of an open-type diaper as illustrated but also in the form of pants-type diaper in which the front waist region 6 and the rear waist region 7 have been connected to each other along transversely opposite side edge portions thereof by means of welding or the other appropriate technique.

In the disposable diaper according to this invention, the superabsorbent polymer particles and the fluff pulp fibers are disposed between the top- and backsheets in the vicinity of the bottoms of the grooves formed on the core and consequently there is no problem that the surface area of the core available to absorb the body fluids might be reduced by the presence of the grooves.

Along the respective grooves formed on the core, the superabsorbent polymer particles are joined integrally with the absorbent and diffusive sheet which is, in turn, in close contact with the inner surface of the topsheet. This arrangement can reliably avoid the anxiety that the superabsorbent polymer particles might be scattered away far from the vicinity of the grooves even when the grooves are deformed under a wearer's body weight exerted thereon.

## Claims

1. A disposable diaper (1) comprising:
a liquid-pervious topsheet (2);
a liquid-impervious backsheet (3);
a liquid-absorbent core (4) covered with an absorbent and diffusive sheet (33) and disposed between said topsheet and said backsheet;
said core being formed on a side of said topsheet with at least one groove (15) depressed in a direction from a side of said topsheet toward a side of said backsheet and said groove has a bottom (34) and side walls (36) both covered with said topsheet;
said core containing water-absorbent fibers and superabsorbent polymer particles (32);
said water-absorbent fibers and superabsorbent polymer particles being partially disposed between said topsheet and said backsheet along said bottom of said groove; and
said water-absorbent fibers forming a thin layer (41) in close contact with said absorbent and diffusive sheet (33) along the bottom (34) and side walls (36) of the groove (15), the water-absorbent fibers in the thin layer having a density higher than the density of said water-absorbent fibers around a depth of said core.

2. A disposable diaper as claimed in Claim 1, wherein said superabsorbent polymer particles are joined integrally with the absorbent and diffusive sheet covering said core along said bottom and side walls of said groove.

3. A disposable diaper as claimed in Claim 1 or Claim 2, wherein said absorbent and diffusive sheet is joined to said topsheet along said bottom and side walls of said groove by means of adhesive.

4. A disposable diaper as claimed in any preceding claim, wherein said water-absorbent fibers are fluff pulp fibers.

5. A disposable diaper as claimed in any preceding claim, wherein a distribution density of said superabsorbent polymer particles gradually increases in a thickness direction of said core from said topsheet toward said backsheet.

6. A disposable diaper as claimed in any preceding claim, wherein said disposable diaper has a front waist region (6), a rear waist region (7) and a crotch region (8) extending between these two waist regions and wherein said groove extends in a longitudinal direction of said diaper.

7. A disposable diaper as claimed in Claim 6, wherein said crotch region is formed along transversely opposite side edge portions thereof with said grooves, respectively, extending in said longitudinal direction in parallel to each other.

8. A disposable diaper as claimed in Claim 7, wherein a distribution density of said superabsorbent polymer particles in said core is higher between said grooves extending in parallel to each other than outside said grooves.

## Patentansprüche

1. Wegwerfwindel (1) mit:
einer flüssigkeitsdurchlässigen oberen Lage (2);
einer flüssigkeitsundurchlässigen hinteren Lage (3);
einem flüssigkeitsabsorbierenden Kern (4), der mit einer absorbierenden und diffusiven Lage (33) überzogen und zwischen der oberen und der hinteren Lage angeordnet ist;
wobei der Kern auf einer Seite der oberen Lage mit wenigstens einer Vertiefung (15) ausgebildet ist, die in einer Richtung von einer Seite der oberen Lage zu einer Seite der hinteren Lage hin eingedrückt ist, und die Vertiefung einen Boden (34) und Seitenwände (36) aufweist, die jeweils mit der oberen Lage überzogen sind;
wobei der Kern wasserabsorbierende Fasern und superabsorbierende Polymerteilchen (32) enthält;
wobei die wasserabsorbierenden Fasern und die superabsorbierenden Polymerteilchen teilweise zwischen der oberen und der hinteren Lage längs des Vertiefungsbodens angeordnet sind; und
die wasserabsorbierenden Fasern eine dünne Schicht (41) in engem Kontakt mit der absorbierenden und diffusiven Lage (33) längs des Bodens (34) und der Seitenwände (36) der Vertiefung (15) bilden, und die wasserabsorbierenden Fasern in der dünnen Schicht eine höhere Dichte aufweisen als die wasserabsorbierenden Fasern um die Kerntiefe.

2. Wegwerfwindel nach Anspruch 1, wobei die superabsorbierenden Polymerteilchen einstückig mit der absorbierenden und diffusiven Lage verbunden sind, die den Kern längs des Bodens und der Seitenwände der Vertiefung überzieht.

3. Wegwerfwindel nach Anspruch 1 oder 2, wobei die absorbierende und diffusive Lage längs des Bodens und der Seitenwände der Vertiefung mittels eines Klebstoffs mit der oberen Lage verbunden ist.

4. Wegwerfwindel nach einem der vorstehenden Ansprüche, wobei die wasserabsorbierenden Fasern Fluffzellstoff-Fasern sind.

5. Wegwerfwindel nach einem der vorstehenden Ansprüche, wobei eine Verteilungsdichte der superabsorbierenden Polymerteilchen graduell in einer Dickenrichtung des Kerns von der oberen Lage zu der hinteren Lage ansteigt.

6. Wegwerfwindel nach einem der vorstehenden Ansprüche, wobei die Wegwerfwindel einen vorderen Taillenbereich (6), einen hinteren Taillenbereich (7) und einen zwischen den zwei Taillenbereichen verlaufenden Schrittbereich (8) aufweist, und wobei die Vertiefung in einer Längsrichtung der Windel verläuft.

7. Wegwerfwindel nach Anspruch 6, wobei der Schrittbereich längs seiner transversal entgegengesetzten Seitenkantenbereiche jeweils mit den Vertiefungen ausgebildet ist, die in der Längsrichtung parallel zueinander verlaufen.

8. Wegwerfwindel nach Anspruch 7, wobei eine Verteilungsdichte der superabsorbierenden Polymerteilchen in dem Kern zwischen den parallel zueinander verlaufenden Vertiefungen größer ist als außerhalb der Vertiefungen.

## Revendications

1. Couche-culotte jetable (1) comprenant:
une feuille supérieure (2) perméable aux liquides;
une feuille arrière (3) imperméable aux liquides;
un noyau (4) absorbant les liquides recouvert par une feuille absorbante et diffusive (33), et disposé entre ladite feuille supérieure et ladite feuille arrière;
ledit noyau comportant, sur un côté tourné vers ladite feuille supérieure, au moins une rainure (15) enfoncée dans une direction partant d'un côté tourné vers ladite feuille supérieure vers un côté tourné vers ladite feuille arrière, et ladite rainure incluant un fond (34) et des parois latérales (36) qui sont tous recouverts par ladite feuille arrière;
ledit noyau contenant des fibres absorbant l'eau et des particules (32) de polymères superabsorbants;
lesdites fibres absorbant l'eau et lesdits particules de polymères superabsorbants étant disposées partiellement entre ladite feuille supérieure et ladite feuille arrière le long dudit fond de ladite rainure; et
lesdites fibres absorbant l'eau formant une couche mince (41) en contact étroit avec ladite feuille absorbante et diffusive (33) le long du fond (34) et des parois latérales (36) de la rainure (15), la densité des fibres absorbant l'eau contenues dans la couche mince étant plus élevée que la densité desdites fibres absorbant l'eau disposées dans l'épaisseur dudit noyau autour de ladite ou desdites rainure(s).

2. Couche-culotte jetable selon la revendication 1, dans laquelle lesdites particules de polymères superabsorbants sont jointes d'un seul tenant avec la feuille absorbante et diffusive recouvrant ledit noyau le long dudit fond et desdites parois latérales de ladite rainure.

3. Couche-culotte jetable selon la revendication 1 ou la revendication 2, dans laquelle ladite feuille absorbante et diffusive est jointe à ladite feuille supérieure au moyen d'un adhésif le long dudit fond et desdites parois latérales de ladite rainure.

4. Couche-culotte jetable selon l'une quelconque des revendications précédentes, dans laquelle lesdites fibres absorbant l'eau sont des fibres de pulpe de duvet de peluchage.

5. Couche-culotte jetable selon l'une quelconque des revendications précédentes, dans laquelle la densité de la répartition desdites particules de polymères superabsorbants est croissante, dans une direction d'épaisseur dudit noyau, à partir de ladite feuille supérieure vers ladite feuille arrière.

6. Couche-culotte jetable selon l'une quelconque des revendications précédentes, dans laquelle ladite couche-culotte inclut une région de ceinture avant (6), une région de ceinture arrière (7) et une région d'entrejambe (8) s'étendant entre ces deux régions de ceinture, et dans laquelle ladite rainure s'étend dans une direction longitudinale de ladite couche-culotte.

7. Couche-culotte jetable selon la revendication 6, dans laquelle lesdites rainures s'étendant parallèlement les unes aux autres dans ladite direction longitudinale sont respectivement formées, dans ladite région d'entrejambe, le long de parties de bord latéraux transversalement opposés de cette région.

8. Couche-culotte jetable selon la revendication 7, dans laquelle la densité de la répartition desdites particules de polymères superabsorbants dans ledit noyau est plus élevée entre lesdites rainures s'étendant parallèlement les unes aux autres qu'à l'extérieur desdites rainures.
